# EUROPEAN PATENT APPLICATION

(11) **EP 4 778 508 A1**
(43) Date of publication of application: **22.07.2026**
(21) Application number: 26460003.2
(22) Date of filing: 15.01.2026
(51) Int. Cl.: A61K 8/60, A61K 8/64, A61K 8/67, A61K 8/9789, A61K 8/98, A61Q 1/10, A61Q 7/00

(54) **COSMETIC COMPOSITION FOR STIMULATING EYE HAIR GROWTH**

(30) Priority: 15.01.2025 EP 25460003
(71) Applicant: Zaklady Farmaceutyczne Polpharma S.A., 83-200 Starogard Gdanski (PL)
(72) Inventor: Grochal, Katarzyna, 05-500 Piaseczno (PL)
(74) Representative: Kondrat, Mariusz

(57) **Abstract**

The present invention relates to a cosmetic aqueous composition comprising a combination of compounds for improving hair growth when applied to eyelashes and/or eyebrows.

## Description

The present invention relates to a cosmetic aqueous composition comprising a combination of compounds for improving hair growth when applied to eyelashes and/or eyebrows.

### BACKGROUND

Eyelashes, in addition to their contribution to appearance, play a functional role in protecting sensitive ocular structures from the entry of foreign particles. Also, eyelash prominence has a recognised aesthetic function, with long, thick eyelashes being considered a desirable physical attribute with a positive psychological effect.

However, frequent use by women of mascaras, eyelash curlers, and aggressive makeup removal sessions accelerate the loss and reduction in the number, condition and appearance of eyelashes. In addition, with age, there is a decrease in the pigmentation of the eyelashes, as well as of other keratin fibers, making them less visible.

Eyebrows are also useful in preventing the entry of sweat and rain in the eye, but eyebrows main function is at present consider its role in communication. In the last years, the fashion around eyebrows aesthetics demands a fuller appearance.

As a result, there is a need in the cosmetics industry for products that can improve the condition of eyelashes and eyebrows, reduce or slow down eyelash loss, and induce or stimulate their growth and increase their natural pigmentation.

Certain therapeutic agents are known to induce eyelash growth, such as latanoprost or bimatoprost, which increase eyelash growth and also increase the natural pigmentation of eyelashes. However, it is desirable to achieve the eyelash growth effect with a composition lacking prostaglandin analogues, to avoid prejudicial side effects.

The present invention provides a cosmetic aqueous composition lacking any prostaglandin analogue, which is effective in improving hair growth when applied to eyelashes and/or eyebrows, with a regenerating and/or nourishing effect.

### SUMMARY OF THE INVENTION

A first aspect of the present invention relates to a cosmetic aqueous composition comprising troxerutin, *Isochrysis galbana* extract, myristoyl pentapeptide-17, N-Biotinyl-Gly-His-Lys, *Caesalpinia spinosa* gum and biotin.

A second aspect of the present invention relates to the cosmetic aqueous composition of the first aspect for use in improving hair growth when applied to eyelashes and/or eyebrows.

A third aspect of the present invention relates to the cosmetic aqueous composition of the first aspect for use in regenerating and/or nourishing eyelashes and/or eyebrows.

### DETAILED DESCRIPTION OF THE INVENTION

The inventors of the present invention have found that the composition of the first aspect is surprisingly useful in improving hair growth when applied to eyelashes and/or eyebrows, with a regenerating and/or nourishing effect visible after only two weeks of once daily administration.

In a preferred embodiment of the first aspect of the present invention, part of the *Caesalpinia spinosa* gum is hydrolyzed.

In a preferred embodiment of the first aspect of the present invention, the cosmetic aqueous composition further comprises at least one of glycerine, dipropylene glycol, panthenol, pentylene glycol, hydroxyethylcellulose, sodium hydroxide, sodium nitrate, ethylhexylglycerin, caprylhydroxamic acid, potassium sorbate, sodium benzoate, 1,2-hexanediol, caprylyl glycol and benzoic acid. In a preferred embodiment, the cosmetic aqueous composition further comprises glycerine, dipropylene glycol, panthenol, pentylene glycol, hydroxyethylcellulose, sodium hydroxide, sodium nitrate, ethylhexylglycerin, caprylhydroxamic acid, potassium sorbate, sodium benzoate, 1,2-hexanediol. caprylyl glycol and benzoic acid.

In a preferred embodiment of the first aspect of the present invention, the cosmetic aqueous composition comprises glycerine, dipropylene glycol, panthenol, pentylene glycol, troxerutin, *Isochrysis galbana* extract, myristoyl pentapeptide-17, N-Biotinyl-Gly-His-Lys, hydrolyzed *Caesalpinia spinosa* gum, *Caesalpinia spinosa* gum, biotin, hydroxyethylcellulose, sodium hydroxide, sodium nitrate, ethylhexylglycerin, caprylhydroxamic acid, potassium sorbate, sodium benzoate, 1,2-hexanediol, caprylyl glycol, benzoic acid and water. In a preferred embodiment, the cosmetic aqueous composition of the present invention consists of glycerine, dipropylene glycol, panthenol, pentylene glycol, troxerutin, *Isochrysis galbana* extract, myristoyl pentapeptide-17, N-Biotinyl-Gly-His-Lys, hydrolyzed *Caesalpinia spinosa* gum, *Caesalpinia spinosa* gum, biotin, hydroxyethylcellulose, sodium hydroxide, sodium nitrate, ethylhexylglycerin, caprylhydroxamic acid, potassium sorbate, sodium benzoate, 1,2-hexanediol, caprylyl glycol, benzoic acid and water.

In a preferred embodiment of the first aspect of the present invention, the cosmetic aqueous composition of the first aspect comprises 0.02 - 1.00 % w/v troxerutin, 0.2 - 2 % w/v Isochrysis Galbana extract, 0.1 - 10.0 % w/v myristoyl Pentapeptide-17, 0.2 - 5.0 % w/v N-Biotinyl-Gly-His-Lys, 0.2 - 5 % w/v hydrolyzed Caesalpinia Spinosa gum, 0.2 - 5 % w/v Caesalpinia Spinosa gum and/or 0.001 - 0.100 % w/v biotin. The % w/v is with respect to the total weight and volume of the cosmetic aqueous composition.

In a preferred embodiment of the first aspect of the present invention, the cosmetic aqueous composition of the first aspect comprises 2.5-20 % w/v glycerine, 0.2 - 10 % w/v dipropylene glycol, 0.2 - 10 % w/v ethylhexylglycerin, 0.2 - 10 % w/v caprylhydroxamic acid, 0.2 - 10 % w/v panthenol, 0.001 - 0.100 % w/v biotin, 0.02 - 1.00 % w/v troxerutin, 0.1 - 10.0 % w/v myristoyl pentapeptide-17, 0.2 - 5.0 % w/v N-Biotinyl-Gly-His-Lys, 0.2 - 5 % w/v hydrolyzed *Caesalpinia spinosa* gum, 0.2 - 5 % w/v *Caesalpinia spinosa* gum, 0.2 - 2 % w/v potassium sorbate, 0.2 - 2 % w/v sodium benzoate, 0.2 - 2 % w/v pentylene glycol, 0.2 - 2 % w/v *Isochrysis galbana* extract, 0.2 - 2 % w/v hydroxyethylcellulose, 0.2 - 2 % w/v sodium nitrate, 0.02 - 0.20 % w/v 1,2-hexanediol, 0.02 - 0.20 % w/v caprylyl glycol, 0.002 - 0.020 % w/v benzoic acid, sodium hydroxide sufficient for pH adjustment, and water until 100% w/v.

In a preferred embodiment of the first aspect of the present invention, the cosmetic aqueous composition of the first aspect consists of 2.5-20 % w/v glycerine, 0.2 - 10 % w/v dipropylene glycol, 0.2 - 10 % w/v ethylhexylglycerin, 0.2 - 10 % w/v caprylhydroxamic acid, 0.2 - 10 % w/v panthenol, 0.001 - 0.100 % w/v biotin, 0.02 - 1.00 % w/v troxerutin, 0.1 - 10.0 % w/v myristoyl pentapeptide-17, 0.2 - 5.0 % w/v N-Biotinyl-Gly-His-Lys, 0.2 - 5 % w/v hydrolyzed *Caesalpinia spinosa* gum, 0.2 - 5 % w/v *Caesalpinia spinosa* gum, 0.2 - 2 % w/v potassium sorbate, 0.2 - 2 % w/v sodium benzoate, 0.2 - 2 % w/v pentylene glycol, 0.2 - 2 % w/v *Isochrysis galbana* extract, 0.2 - 2 % w/v hydroxyethylcellulose, 0.2 - 2 % w/v sodium nitrate, 0.02 - 0.20 % w/v 1,2-hexanediol, 0.02 - 0.20 % w/v caprylyl glycol, 0.002 - 0.020 % w/v benzoic acid, sodium hydroxide sufficient for pH adjustment, and water until 100% w/v.

In a preferred embodiment of the first aspect of the present invention, the cosmetic aqueous composition of the first aspect is packaged in a container comprising an applicator attached to a removable cap. The container includes a reservoir containing the composition and a removable cap for closing the reservoir, comprising a sleeve or handle for a brush carried at the end of a rod joined to the cap. In a preferred embodiment, the brush's shape is linear, and its disposition is parallel to the sleeve or handle and located at the end of said sleeve, instead of having radial bristles in a helical arrangement. In the closed position of the applicator set, the rod and brush plunge into the reservoir. When the rod is withdrawn from the reservoir, a certain quantity of composition is picked up on the brush and can be applied to the eyelashes and eyebrows. Preferably, the brush penetrates to the interior of the reservoir via a substantially circular opening bordered by a flexible lip, the role of which is to exert a wringing or squeegee action on the bristles of the brush and eliminate the excess product picked up by the brush from the inside of the reservoir. In these applicators, the quantity of composition picked up with the aid of a brush is always about the same. In a preferred embodiment, the container comprises between 1 ml and 10 ml of the cosmetic aqueous composition. In a preferred embodiment, the container comprises between 2 ml and 5 ml of the cosmetic aqueous composition. In a preferred embodiment, the container comprises about 3 ml of the cosmetic aqueous composition.

The present invention provides a cosmetic aqueous composition which is effective in improving hair growth when applied to eyelashes and/or eyebrows, with a regenerating and/or nourishing effect. The cosmetic aqueous composition of the present invention has proven to be an active strengthening composition that intensively regenerates and nourishes eyelashes and eyebrows. It densifies and volumizes the lashes, improves condition and prevents breakage and eyelash loss. Upon daily administration, eyelashes and eyebrows appear thicker, stronger, shinier and more flexible. Thus, it helps to beautifully and naturally define the eyes with first results being visible after just 2 weeks of daily administration.

In a preferred embodiment of the second and third aspects of the present invention, the cosmetic aqueous composition is applied once daily. Preferably, the administration is done once a day before going to bed. Using the applicator, it is possible to apply a small amount in one swipe to clean (washed) and dry eyelid skin, at the root of the eyelashes (bulb). The product can be applied to both the upper and lower eyelid and eyebrows and can be used regularly.

In any of the aspects of the invention, the present invention refers also to the embodiments where the expression "comprises" or "comprising" means "consists of" or "consisting of". The present invention includes any combination of the different embodiments and preferred embodiments disclosed herein for any of the aspects of the invention.

As used herein, the term "about" means plus/minus 10 %.

An aqueous cosmetic composition with 2.5-20 % w/v glycerine, 0.2 - 10 % w/v dipropylene glycol, 0.2 - 10 % w/v ethylhexylglycerin, 0.2 - 10 % w/v caprylhydroxamic acid, 0.2 - 10 % w/v panthenol, 0.001 - 0.100 % w/v biotin, 0.02 - 1.00 % w/v troxerutin, 0.1 - 10.0 % w/v myristoyl pentapeptide-17, 0.2 - 5.0 % w/v N-Biotinyl-Gly-His-Lys, 0.2 - 5 % w/v hydrolyzed *Caesalpinia spinosa* gum, 0.2 - 5 % w/v *Caesalpinia spinosa* gum, 0.2 - 2 % w/v potassium sorbate, 0.2 - 2 % w/v sodium benzoate, 0.2 - 2 % w/v pentylene glycol, 0.2 - 2 % w/v *Isochrysis galbana* extract, 0.2 - 2 % w/v hydroxyethylcellulose, 0.2 - 2 % w/v sodium nitrate, 0.02 - 0.20 % w/v 1,2-hexanediol, 0.02 - 0.20 % w/v caprylyl glycol, 0.002 - 0.020 % w/v benzoic acid, sodium hydroxide sufficient for pH adjustment, and water until 100% w/v, was prepared and the following study was performed to evaluate the tolerance of eyes and skin of the product and assess its properties and efficiency after regular application by a panel of 20 subjects. Pre and post eye examinations were be performed by an ophthalmologist. The product was applied, under normal conditions of use, by the test subjects at home for 84 days. The test subject was used as own control.

Description of the subjects:

General inclusion criteria:
- Healthy subject.
- Sign an informed consent to participate in the study, were informed about the purpose of the study, the manner of its conduct and the possible side effects.
- Skin without irritation and changes requiring pharmacological treatment.
- Phototype: I - IV on Fitzpatrick scale.
- Cooperative subject, aware of the necessity and duration of controls.
- Subject free of any clinically significant ophthalmic findings such as: external ocular diseases or infections of the eyes and/or eyelids, glaucoma, cataracts, corneal or maculopathies that could have progressed during the course of this study.

Specific inclusion criteria:
- Number of subjects: 20 subjects
- Gender: Women
- Age: 18-50
- Skin type: All
- Others: The subjects with eyelash extensions (1 person), after eyelash treatments: henna (1 person), lamination (1 person)

Non-inclusion criteria:
- Subjects who use any treatment on the studied zone.
- Pregnant or breastfeeding woman or woman planning a pregnancy during the study.
- Subject presenting a pathology on the studied zone (ex. severe acne, scars).
- History of drug or sun hypersensitivity, recurrent dermatological diseases or recent sunburn.
- Use of topical or systemic treatment during the previous weeks liable to interfere with the assessment of the tolerance of each studied product.
- Subject enrolled in another study during the study period (concerning the studied zone).
- Subject considered to be likely not compliant to the protocol.

Informed consent:
After an explanation of the protocol, reasons for the study, possible associated risks and potential benefits of the treatment each subject signed an informed consent form before starting the study.

The qualified subjects received the product, a daily log and were obliged to:
- regular use of the product according to the method of use,
- during the test any other products of similar effects must not be used.
- a detailed evaluation of the tested product by using the received questionnaire,
- in case of discomfort or any medicine taken fill the daily log,
- in case of any side effects they should immediately stop using the product and consult specialist.

Skin reactivity, history of atopy and contraception were documented. No medication likely to interfere with the study was allowed during the study; however, if the health state of the subjects justifies some medication (particularly anti-inflammatory drugs), any information relating to this concomitant medication had to be carefully documented in the case report form. Subjects taking concomitant medication likely to interfere with the study and the interpretation of the results had to be excluded.

Trial schedule:

Day 0:
- The subjects come to the laboratory without having applied any product on the studied zone.
- Subjects sign and consent informed forms in duplicate.
- Verification of inclusion and non-inclusion criteria.
- The ophthalmologist examines the study zone.
- Instrumental test - VISIA^{®} length and volume of eyelashes analysis.
- Distribution studied product, daily log and questionnaire.

Days 14, 28, 56:
- The subjects came to the laboratory without having applied any product on the studied zone.
- The ophthalmologist examined the study zone.
- Instrumental test - VISIA^{®} length and volume of eyelashes analysis.
- Collect filled questionnaire.
- Record of possible adverse reactions.

Day 84:
- The subjects came to the laboratory without having applied any product on the studied zone.
- The ophthalmologist examined the study zone.
- Instrumental test - VISIA^{®} length and volume of eyelashes analysis.
- Collecting and checking the daily log.
- Collect filled questionnaire.
- Record of possible adverse reactions.

Ophthalmological examination showed no changes in the anterior segment and the camera protective eye in all subjects participating in the use test. The tested product does not cause burning eyes, stinging, itching, redness, tearing, eye lesions and swollen eyelids at any of the days. On the basis of medical examinations and interviews collected from subjects is found that the product was very well tolerated by the eyes.

Based on the replies by the study subjects to the questionnaire, 91% of the participants confirmed that their eyelashes and eyebrows had become stronger, 86% of the participants confirmed that their eyelashes and eyebrows have become thicker and fuller, 91% of the respondents confirmed that their eyelashes and eyebrows had become visibly refreshed and/or nourished.

Based on the Instrumental test - VISIA^{®} length and volume of eyelashes analysis, the product improves length and volume of eyelashes after 14 days, after 28 days, after 56 and after 84 days of regular use.

Conclusion:

Under these study conditions, after 84 days of regular application it is concluded, that the product:
- was tested under ophthalmologist supervision,
- was very well tolerated by the eyes,
- properties have been confirmed basing on a subjective questionnaire:
   o The product visibly strengthens the lashes/eyebrows.
   o The product lengthens lashes.
   o The product thickens lashes/eyebrows.
   o The product increases the density of lashes/eyebrows.
   o The product visibly reduces the loss of lashes/eyebrows.
   o The product makes growing lashes/eyebrows visibly thicker, longer and stronger.
   o The product visibly stimulates the regrowth of lashes/eyebrows.
   o The product visibly improves the condition of lashes/eyebrows.
   o The product visibly nourishes/regenerates lashes/eyebrows.
   o Lashes/eyebrows become smoother.
   o The product improves the appearance of sparse and thin lashes.
   o Lashes/eyebrows appear fuller. more voluminous.
   o The product helps to achieve a beautiful and natural eye frame.
   o The product makes lashes/eyebrows less brittle.
   o Lashes/eyebrows become flexible.
   o Lashes/eyebrows become shiny, full of lustre.
   o The product restores the healthy appearance of lashes/eyebrows.
   o The product darkens hair.
   o The product packaging is handy.
   o Use of the product reduces the need for costly salon treatments.
- properties have been confirmed basing on instrumental tests:
   o improves the length of eyelashes:
      ■ after 14 days of regular use (average 4%),
      ■ after 28 days of regular use (average 11%),
      ■ after 56 days of regular use (average 18%),
      ■ after 84 days of regular use (average 20%).
   o improves the volume of eyelashes:
      ■ after 14 days of regular use (average 9%),
      ■ after 28 days of regular use (average 9%),
      ■ after 56 days of regular use (average 11%),
      ■ after 84 days of regular use (average 12%).

## Claims

1. Cosmetic aqueous composition comprising troxerutin, *Isochrysis galbana* extract, myristoyl pentapeptide-17, N-Biotinyl-Gly-His-Lys, *Caesalpinia spinosa* gum and biotin.

2. The cosmetic aqueous composition according to claim 1, wherein part of the *Caesalpinia spinosa* gum is hydrolyzed.

3. The cosmetic aqueous composition according to any one of claims 1 or 2, further comprising at least one of glycerine, dipropylene glycol, panthenol, pentylene glycol, hydroxyethylcellulose, sodium hydroxide, sodium nitrate, ethylhexylglycerin, caprylhydroxamic acid, potassium sorbate, sodium benzoate, 1,2-hexanediol, caprylyl glycol and benzoic acid.

4. The cosmetic aqueous composition according to any one of claims 1 to 3, consisting of glycerine, dipropylene glycol, panthenol, pentylene glycol, troxerutin, *Isochrysis galbana* extract, myristoyl pentapeptide-17, N-Biotinyl-Gly-His-Lys, hydrolyzed *Caesalpinia spinosa* gum, *Caesalpinia spinosa* gum, biotin, hydroxyethylcellulose, sodium hydroxide, sodium nitrate, ethylhexylglycerin, caprylhydroxamic acid, potassium sorbate, sodium benzoate, 1,2-hexanediol, caprylyl glycol, benzoic acid and water.

5. The cosmetic aqueous composition according to any one of claims 1 to 4, comprising 0.02 - 1.00 % w/v troxerutin, 0.2 - 2 % w/v Isochrysis Galbana extract, 0.1 - 10.0 % w/v myristoyl Pentapeptide-17, 0.2 - 5.0 % w/v N-Biotinyl-Gly-His-Lys, 0.2 - 5 % w/v hydrolyzed Caesalpinia Spinosa gum, 0.2 - 5 % w/v Caesalpinia Spinosa gum and/or 0.001 - 0.100 % w/v biotin.

6. The cosmetic aqueous composition according to any one of claims 1 to 5 comprising:
2.5-20 % w/v glycerine,
0.2 - 10 % w/v dipropylene glycol,
0.2 - 10 % w/v ethylhexylglycerin,
0.2 - 10 % w/v caprylhydroxamic acid,
0.2 - 10 % w/v panthenol,
0.001 - 0.100 % w/v biotin,
0.02 - 1.00 % w/v troxerutin,
0.1 - 10.0 % w/v myristoyl pentapeptide-17,
0.2 - 5.0 % w/v N-Biotinyl-Gly-His-Lys,
0.2 - 5 % w/v hydrolyzed *Caesalpinia spinosa* gum,
0.2 - 5 % w/v *Caesalpinia spinosa* gum,
0.2 - 2 % w/v potassium sorbate,
0.2 - 2 % w/v sodium benzoate,
0.2 - 2 % w/v pentylene glycol,
0.2 - 2 % w/v *Isochrysis galbana* extract,
0.2 - 2 % w/v hydroxyethylcellulose,
0.2 - 2 % w/v sodium nitrate,
0.02 - 0.20 % w/v 1,2-hexanediol,
0.02 - 0.20 % w/v caprylyl glycol,
0.002 - 0.020 % w/v benzoic acid
sodium hydroxide sufficient for pH adjustment, and
water until 100% w/v.

7. The cosmetic aqueous composition according to any one of claims 1 to 6, consisting of:
2.5-20 % w/v glycerine,
0.2 - 10 % w/v dipropylene glycol,
0.2 - 10 % w/v ethylhexylglycerin,
0.2 - 10 % w/v caprylhydroxamic acid,
0.2 - 10 % w/v panthenol,
0.001 - 0.100 % w/v biotin,
0.02 - 1.00 % w/v troxerutin,
0.1 - 10.0 % w/v myristoyl pentapeptide-17,
0.2 - 5.0 % w/v N-Biotinyl-Gly-His-Lys,
0.2 - 5 % w/v hydrolyzed *Caesalpinia spinosa* gum,
0.2 - 5 % w/v *Caesalpinia spinosa* gum,
0.2 - 2 % w/v potassium sorbate,
0.2 - 2 % w/v sodium benzoate,
0.2 - 2 % w/v pentylene glycol,
0.2 - 2 % w/v *Isochrysis galbana* extract,
0.2 - 2 % w/v hydroxyethylcellulose,
0.2 - 2 % w/v sodium nitrate,
0.02 - 0.20 % w/v 1,2-hexanediol,
0.02 - 0.20 % w/v caprylyl glycol,
0.002 - 0.020 % w/v benzoic acid,
sodium hydroxide sufficient for pH adjustment, and
water until 100% w/v.

8. The cosmetic aqueous composition according to any one of claims 1 to 7 packaged in a container comprising an applicator attached to a removable cap.

9. The cosmetic aqueous composition according to claim 8, wherein the container comprises between 1 ml and 10 ml of the cosmetic aqueous composition.

10. The cosmetic aqueous composition according to any one of claims 1 to 9, for use in improving hair growth when applied to eyelashes and/or eyebrows.

11. The cosmetic aqueous composition according to any one of claims 1 to 9, for use in regenerating and/or nourishing eyelashes and/or eyebrows.

12. The cosmetic aqueous composition for use according to any one of claims 10 or 11, wherein said composition is applied once daily.
